# EUROPÄISCHE PATENTSCHRIFT

(11) **EP 1 227 794 B1**
(45) Veröffentlichungstag und Bekanntmachung des Hinweises auf die Patenterteilung: **30.11.2005**
(21) Anmeldenummer: 00976017.4
(22) Anmeldetag: 09.11.2000
(51) Int. Cl.: A61K 9/127

(54) **VERFAHREN ZUR VERKAPSELUNG VON PROTEINEN ODER PEPTIDEN IN LIPOSOMEN**
METHOD FOR ENCAPSULATING PROTEINS OR PEPTIDES IN LIPOSOMES
PROCEDE D'ENCAPSULAGE DE PROTEINES OU DE PEPTIDES DANS DES LIPOSOMES

(30) Priorität: 09.11.1999 DE 19954843
(43) Veröffentlichungstag der Anmeldung: 07.08.2002
(73) Patentinhaber: Novosom GmbH, 06120 Halle (DE)
(72) Erfinder: PANZNER, Steffen, 06108 Halle (DE)
(74) Vertreter: Ziebig, Marlene
(86) Internationale Anmeldenummer: PCT/EP2000/011079
(87) Internationale Veröffentlichungsnummer: WO 2001/034115

(56) Entgegenhaltungen:
- US-A- 4 342 826
- US-A- 4 933 121
- DATABASE WPI Week 198226 Derwent Publications Ltd., London, GB; AN 1982-53654e XP002167845 & JP 57 082310 A (TANABE SEIYAKU)

## Beschreibung

Die Erfindung betrifft ein neues Verfahren zur Verkapselung von Proteinen oder Peptiden in Liposomen, das gute Einschlussraten > 30 % aufweist.

Liposomen können auf unterschiedliche Weise hergestellt und mit Fremdstoffen beladen werden. Eine zusammenfassende Darstellung findet sich unter anderem bei R.R.C. New, Liposomes - A practical approach. IRL Press 1990.

In der Vergangenheit wurde eine große Anzahl von Studien durchgeführt, bei denen klassische, niedermolekulare Arzneistoffe in liposomaler Form appliziert wurden. Solche Formulierungen sind mittlerweile kommerziell erhältlich. In jüngerer Zeit rücken Proteine und andere komplexe wirkstoffmoleküle in das Zentrum der Aufmerksamkeit. Sie bieten gegenüber den konventionellen Wirkstoffen Vorteile insbesondere durch ihre hohe Spezifität und ihren authentischen Charakter. Nachteilig bei der Verwendung der Mehrzahl von biologisch wirksamen Proteinen oder Peptiden ist deren Immunogenität und ihr allergenes Potential, wenn sie ungeschützt in die Blutbahn gelangen. Darüber hinaus sind diese Proteine dort dem Angriff abbauender Enzyme ausgesetzt. Solche Nachteile lassen sich durch die Verwendung liposomaler Träger reduzieren. Eine wichtige Voraussetzung bei der Verwendung solcher liposomalen Formulierungen ist ein hohes Verhältnis von eingeschlossenem Material zu liposomalem Träger. Dieses Verhältnis ist bei den bekannten Formulierungen hoch, wenn (i) das Cargo - also die einzuschließende Substanz - in sehr hoher Konzentration vorliegt und (ii) große, unilamellare Liposomen entstehen, da diese ein günstiges Verhältnis von Oberfläche zu eingeschlossenem Volumen besitzen. Eine Forderung besteht darin, möglichst viel des angebotenen Cargos in die Liposomen einzuschließen. Das ist durch eine Erhöhung der Lipidkonzentration bis zu einem gewissen Punkt möglich, diese Maßnahme läuft aber dem Bestreben nach Minimierung der eingesetzten Lipidmenge zuwider.

Nach dem bekannten Stand der Technik wird ein hohes Verhältnis von Cargo zu liposomalem Träger immer dann erreicht, wenn die membranbildenden Substanzen, wie z.B. Lipide, und das einzuschließende Material in sehr hohen Konzentrationen vorliegen und die Menge des wässrigen Lösungsmittels soweit als möglich reduziert wird. Bisher werden liposomale Formulierungen mit hoher Einschlusseffizienz wie folgt hergestellt:

Nach einer Methode von Deamer (Method for encapsulating Materials into Liposomes US-A 4.515.736, 1985) werden zunächst leere Liposomen hergestellt und zusammen mit dem einzuschließenden Stoff eingetrocknet. Dabei kommt es zum Aufbrechen der bis dahin geschlossenen Membranschicht und zum Konzentrationsausgleich zwischen Außenmedium und Binnenraum. Durch eine sich anschließende Rehydratisierung entstehen sehr große liposomale Aggregate, die durch Hochdruckextrusion wieder zerkleinert werden.

Nach Barenholz et al. (A Method for high loading of vesicles hith biopolymeric substances. WO 95/04524, 1993) wird zuerst eine hochkonzentrierte liposomale Paste erzeugt, der dann der einzuschließende Stoff und ein organisches, wasserlösliches Lösungsmittel zu Erhöhung der Permeabilität der Liposomen zugesetzt wird, wodurch sich ein Konzentrationsausgleich zwischen Außenmedium und Binnenraum erreichen lässt.

Andere beschriebene Verfahren (Papahadjopoulos et al., Method of encapsulating biologically active materials in lipid vesicles, US-A 4.235.871, 1980; Martin et al., High-encapsulation Liposome Processing Method. US-A 4.752.425, 1988) erreichen einen Einschluss von Cargomolekülen bei der Injektion einer nicht wassermischbaren, organischen Lösung von Lipiden in eine wäßrige Lösung des einzuschließenden Stoffes. Wird das organische Lösungsmittel des Lipids entsprechend seiner Zuführung abgedampft, so lässt sich dieser Prozess bis zur Erreichung sehr hoher Lipidkonzentrationen fortführen.

US-A 4.342.826 beschreibt ein Verfahren zur immunspezifischen Lyse von Liposomen für den Nachweis von Immunreaktionen

Alle diese bekannten Techniken beruhen auf einem lediglich passiven Konzentrationsausgleich zwischen dem Außenmedium und dem Binnenraum der Liposomen. Die erreichbare Wirkstoffkonzentration im Binnenraum kann also maximal der in der Ausgangslösung vorhandenen Konzentration entsprechen. Es wird keine Aufkonzentrierung erreicht.

Zudem haben diese Verfahren den Nachteil, dass sämtlich große, multi- oder oligolamellare Liposomen erzeugt werden. Bei der anschließenden Zerkleinerung wird zwangsläufig ein Teil des eingeschlossenen Materials wieder freigesetzt.

Ein passiver Einschlussmechanismus durch Verteilung des gelösten Stoffes zwischen Binnenraum und Außenmedium ist in seiner Einschlusseffizienz ganz erheblich von der Größe und Lamellarität der gebildeten Strukturen abhängig. Bei gleichbleibender Lipidkonzentration geht die Einschlusseffizienz mit kleiner werdenden Teilchen oder mit steigender Lamellarität schnell zurück.

Ein weiterer Nachteil der bekannten Methoden ist, dass ein hoher Einschluss des Cargos über Liposomen mit einem großen Durchmesser realisiert wird. Kleine Liposomen können deshalb bisher nicht mit hohen Einschlussraten beladen werden. Bei Verfahren nach dem Stand der Technik ist die Beladungseffizienz durch den Anteil des Innenvolumens der Liposomen am Gesamtvolumen der Flüssigkeit vorgegeben. Dieses Innenvolumen sinkt bei kleinen Liposomen sehr schnell ab, so dass unterhalb einem Durchmesser von 200 nm ein solches Verfahren sehr uneffizient und unwirtschaftlich wird. Bei einer Lipidkonzentration von 10 mg/ml beträgt das Innenvolumen von Liposomen beispielsweise bei einem Durchmesser von 4000 nm nahezu 100%, bei einem Durchmesser von 2000 nm ca. 50 %, bei einem Durchmesser von 1000 nm ca. 30%, bei einem Durchmesser von 500 nm ca. 15% und bei einem Durchmesser von 200 nm ca. 5-7%. Der Anteil des Innenvolumens liegt bei Liposomen mit einem Durchmesser von 100 nm bei nur ca. 2,6%, bei kleineren Strukturen noch weiter darunter.

Die Einschlusseffizienz lässt sich bisher linear außerdem durch die Erhöhung der Lipidmenge steigern, diese ist jedoch nicht unbegrenzt zu steigern und führt außerdem zu einer Formulierung, bei der mit einer sehr großen Menge an Lipid nur eine geringe Menge an Cargo transportiert wird.

Die beschriebenen Verfahren enthalten allesamt Schritte, die geeignet sind, die biologische Aktivität nachteilig zu beeinflussen, wie z.B. durch Lyophilisation oder durch Zugabe von organischen Lösungsmitteln, seien sie wassermischbar oder auch nicht. Weiterhin ist nachteilig, dass die Liposomen sehr groß gewählt werden, um möglichst viel Cargo einzuschließen. Große Liposomen sind jedoch unter bestimmten Bedingungen instabiler und zirkulieren daher im Blut nur eine geringe Zeit. Weiterhin sind große Liposomen in der Regel nicht in der Lage, biologische Barrieren, wie z.B. Membranen von Blutgefäßen, zu durchdringen, um direkt an den Zielort zu gelangen.

Liposomen können beispielsweise bei der Krebstherapie eingesetzt werden. Die Liposomen müssen hierfür mehrere Voraussetzungen erfüllen. Damit die Liposomen die Kapillaren zum Tumorgewebe durchdringen können, müssen sie entsprechend klein sein. Die Liposomen müssen weiterhin mit großer Effizienz beladen werden und sie müssen lange in Körperflüssigkeiten zirkulieren. Liposomen mit einer Größe von über 500 nm zirkulieren nicht ausreichend lange im Blut und können biologische Barrieren kaum durchdringen. Werden kleinere Liposomen im Bereich von 100 - 200 nm gewählt, ist die Einschlusseffizienz bisher nicht ausreichend.

Aus DE 44 02 867 C1 (Schmidt, P.C.; Karau, C.; Fleck, M.; Walch, H., 1994) ist ein Verfahren zur Herstellung von Liposomen mit darin verkapselten Proteinen bekannt. Dieses Verfahren beruht auf der Herstellung eines dünnnen Lipidfilms, wobei die membranbildenden Stoffe Phosphatidylcholin, Cholesterol, ein Phosphatidylglycerolderivat und Tocopherol im Verhältnis 6:4:1:0,01 in einem geeigneten Lösungsmittel gelöst werden und anschließend das Lösungsmittel abgedampft wird. Wird dieser Film durch eine pharmazeutisch geeignete, das einzuschließende Protein enthaltende Lösung wieder rehydratisiert, so lässt sich ein erheblicher Teil der Proteine in die entstehenden Liposomen einschließen. Nach diesem Verfahren wird durch Zusatz von Phosphatidylglycerol die Einschlusseffizienz von Interferonen auf bis zu 48% erhöht. Diese erhöhte Einschlusseffizienz wird auf sich ausbildende (ionische) Wechselwirkungen zwischen der liposomalen Membran und dem Cargo zurückgeführt. Das Verfahren ist jedoch nur bedingt verallgemeinerbar, in vielen Fällen führt ein solches Vorgehen zur Aggregation unterschiedlich geladener Bestandteile und zur Flockung des Kolloids.

Der Erfindung lag nun die Aufgabe zugrunde, ein alternatives Verfahren für die Verkapselung von Proteinen oder Peptiden in Liposomen bereitzustellen, das die oben genannten Nachteile, insbesondere die Aggregation der Liposomen vermeidet oder aufhebt und hohe Einschlussraten von mindestens 30%, insbesondere bei kleinen Liposomen, gewährleistet.

Erfindungsgemäß wird die Aufgabe durch ein Verfahren gelöst, das dadurch gekennzeichnet ist, dass
a) das zu verkapselnde Protein oder Peptid in einem wässrigen Puffermedium gelöst wird,
b) Lösungen membranbildender und/oder membranständiger Substanzen zugesetzt werden, die ausgewählt werden aus
   - geladenen amphiphilen Verbindungen,
   - thiogruppenhaltigen amphiphilen Verbindungen,
   - amphiphilen Derivaten der Trinitriloessigsäure und/oder der Iminodiessigsäure
   und
   der pH-Wert des wässrigen Puffermediums, so eingestellt wird, dass das Protein oder Peptid eine ionische Bindung zu der entstehenden Membran ausbildet,
   oder
   durch Disulfidbrücken kovalente Bindungen ausgebildet werden,
   oder
   chelatisierende Interaktionen ausgebildet werden,
c) Protein oder Peptid, das sich an der Aussenseite der gebildeten Liposomenmembran befindet, durch Erhöhung der Ionenstärke des Mediums, Veränderung des pH-Wertes, Zugabe von Reduktionsmitteln und/oder lytischen Enzymen abgetrennt wird und
d) die Liposomen aus der Lösung gewonnen werden.

Diese Lösungen sind Gemische aus neutralen membranbildenden und/oder membranständigen Komponenten und weiteren membranbildenden und/oder membranständigen Komponenten, die mit den Proteinen oder Peptiden während des Einschlussvorganges eine reversible Wechselwirkung eingehen, d.h. die Proteine oder Peptide werden während des Einschlussvorganges an diese Verbindungen gebunden.

Nach dem Einschlussvorgang liegen die Proteine oder Peptide an der Außen- und Innenseite der gebildeten Liposomen gebunden vor. Die eingesetzten Membrankomponenten, die mit den Proteinen oder Peptiden eine entsprechende Wechselwirkungen eingehen, werden erfindungsgemäß so ausgewählt, dass sie komplementär zu den Proteinen oder Peptiden sind.

Aufgrund der komplementären Wechselwirkungen zwischen den membranständigen Komponenten und den Proteinen oder Peptiden kommt es unter Umständen zu einer Ausflockung der Liposomen. Überraschend wurde festgestellt, dass diese Ausflockung der Liposomen vermieden bzw. wieder aufgehoben werden kann, wenn in einem weiteren Schritt die Verfahrensbedingungen so gewählt werden, dass die Interaktion zwischen den Komponenten aufgehoben wird. Dadurch werden solche Proteine oder Peptide, die sich an der Außenseite der gebildeten Liposomenmembran befinden, abgetrennt und können einem erneuten Einsatz zugeführt werden. Die gebildeten Liposomen werden aus der Lösung gewonnen.

Bevorzugt ist, dass als membranbildende oder membranständige Substanzen, Substanzen, die mit dem Protein oder Peptid ionische Bindungen, kovalente Bindungen oder Chelatkomplexe ausbilden, in Kombination mit weiteren membranbildenden und/oder membranständigen Substanzen, die keine Bindung mit dem Protein oder Peptid eingehen, eingesetzt werden.

Membranbildende oder membranständige Substanzen, die mit den Proteinen oder Peptiden eine reversible Wechselwirkung eingehen, sind im Sinne der Erfindung Verbindungen, die mit den Proteinen oder Peptiden entweder ionische Bindungen, kovalente Bindungen oder chelatisierende Interaktionen ausbilden. Sie werden in Kombination mit weiteren membranbildenden oder membranständigen Komponenten, die keine Bindung mit den Proteinen oder Peptiden eingehen, eingesetzt. Bevorzugt werden demzufolge Verbindungen eingesetzt, die eine unterschiedliche Ladung zu den Proteinen oder Peptiden aufweisen oder mit ihnen zur Bildung von Disulfidbrücken oder Chelatkomplexen befähigt sind.

Solche membranbildende Substanzen, die mit den Proteinen oder Peptiden eine ionische Bindung eingehen, sind geladene amphiphile Verbindungen bevorzugt Alkylcarbonsäuren, Alkylsulfonsäuren, Alkylamine, Alkylammoniumsalze, Phosphorsäureester mit Alkoholen, natürliche sowie synthetische Lipide, wie Phosphatidylglycerol (PG), Phosphatidylserin (PS), Derivate des Phophatidylethanolamins (PE-Derivate) sowie des Cholesterols, Phosphatidsäure, Phosphatidylinositol, Cardiolipin und/oder geladene Sphingolipide, wie z.B. Sulfatid.

Verbindungen, die mit den Proteinen oder Peptiden Disulfidbrücken ausbilden, sind thiogruppenhaltige Verbindungen, wie z.B. Alkylthiole, Thiocholesterole, Derivate des Phosphatidylethanolamins sowie Derivate von Alkylaminen mit freier Thiolfunktion. Chelatisierende Verbindungen sind amphiphile Derivate der Trinitroessigsäure und/oder der Iminodiessigsäure.

Als neutrale membranbildende Substanzen fungieren an sich bekannte Komponenten wie z.B. Phosphatidylcholin (PC), Phophatidylethanolamin (PE), Steroide, vorzugsweise Cholesterol, komplexe Lipide und/oder neutrale Sphingolipide.

Nach diesem Verfahren werden bevorzugt solche Proteine oder Peptide verkapselt, bei denen die Ausbildung komplementärer Wechselwirkungen mit Liposomen zu einer Flockung des Kolloids führt, Darüber hinaus ist es für weitere Proteine oder Peptide geeignet, deren Präsenz auf der Oberfläche der Liposomen zu unerwünschten biologischen Reaktionen führt.

In einer bevorzugten Ausführungsvariante werden die zu verkapselnden Proteine oder Peptide chemisch vorbehandelt oder unter Nutzung rekombinanter DNA-Techniken so hergestellt, dass die Ausbildung von Disulfidbrücken oder Chelatbindungen ermöglicht wird. Geeignete Methoden dazu sind dem Fachmann bekannt.

Medienzusätze, die eine Interaktion der Proteinen oder Peptide mit den Liposomen stören, müssen vermieden werden. Sollen Disulfidbrücken ausgebildet werden, so stören andere Verbindungen mit freier Thiolfunktion, wenn sie im Überschuss vorliegen. Sollen Chelatbindungen geknüpft werden, so stören andere Chelatbildner oder niedrige pH-Werte, die eine Bildung von Chelaten verhindern.

In einer anderen Verfahrensvariante des erfindungsgemäßen Verfahrens werden die zu verkapselnden Proteine oder Peptide mit einer zu den membranbildenen Substanzen entgegengesetzten Ladung versehen, indem der pH-Vert des wäßrigen Mediums entsprechend eingestellt wird. Es ist bevorzugt, dass der pH-Wert des wässrigen Mediums, in dem das Protein oder Peptid gelöst wird, so eingestellt wird, dass das Protein oder Peptid eine zu den membranbildenden Substanzen entgegengesetzte Ladung aufweist und die Ionenstärke des Mediums hinreichend gering ist, um die Ausbildung dieser Wechselwirkungen zu erlauben. So hat z.B. Hämoglobin einen isoelektrischen Punkt von 6,8. Wird der pH-Wert des Mediums auf einen kleineren Wert eingestellt, so sind die Hämoglobinmoleküle positiv geladen und können mit Liposomen negativer Gesamtladung interagieren. Ist der pH-Wert der Lösung dagegen gößer als der isoelektrische Punkt des Proteins, so hat dieses eine negative Gesamtladung und kann entsprechend mit positiv geladenen Liposomen interagieren. Die Ladung von Partikeln (Proteinen als auch Liposomen) lässt sich unter den gewählten Bedingungen durch die Messung des Zetapotentials bestimmen. Der isoelektrische Punkt von Proteinen kann auch theoretisch berechnet werden.

Nach Bildung der Liposomen werden die an der Außenseite gebundenen Proteine oder Peptide unter Erhalt ihrer Aktivität durch geeignete Verfahren abgetrennt, aus dem Medium entfernt sowie ggf. einem erneuten Einsatz zugeführt.

Im Fall der ionischen Wechselwirkung wird dies z.B. durch Erhöhung der Ionenstärke des Mediums oder durch eine Veränderung des pH-Wertes erreicht. Wurde wie im obigen Beispiel Hämoglobin oberhalb seines isoelektrischen Punktes an positiv geladene Liposomen gebunden, so wird durch Ansäuerung eine Umladung des Proteins erreicht. Hämoglobin und die liposomale Membran sind dann gleichsinnig geladen und stossen einander ab.

Sind die Proteine über Disulfidbrücken an die liposomale Membran gebunden, so werden sie z.B. durch Zugabe von Reduktionsmitteln von der Membran gelöst. Ist das Reduktionsmittel nicht membrangängig, wie etwa Tris-(2-Carboxyethyl-)phosphin, so werden nur die aussen befindlichen Proteine von der Membran gelöst. Membrangängige Reduktionsmittel wie etwa 2-Mercaptoethanol führen dagegen auch im Innern des Liposoms zur Loslösung der Proteine von der Membran.

Sind die Proteine über Chelatbindungen mit der liposomalen Membran verbunden, so wird diese Bindung beispielsweise durch Zusatz anderer Chelatbildner wie etwa der Ethylendiamintetraessigsäure oder Imidazol zum Medium gelöst. Die Chelatbindung wird auch durch eine Ansäurerung des Mediums gelockert.

Schließlich können an der Außenseite befindliche Proteine oder Peptide durch lytische Enzyme, wie z.B. Proteasen oder Peptidasen, abgebaut werden.

Die Gewinnung der Liposomen aus der wäßrigen Lösung erfolgt ebenfalls nach an sich bekannten Techniken so z.B. durch Dialyse, Ultrafiltration, Gelfiltration, Sedimentation oder Flotation.

Es ist bevorzugt, dass das an der Außenseite der Liposomen befindliche Protein oder Peptid unter Erhalt seiner Aktivität durch geeignete Waschschritte abgetrennt, aus dem Medium entfernt und ggf. einem erneuten Einsatz zugeführt wird.

Bevorzugt wird das Protein oder Peptid durch lytische Enzyme abgebaut.

Die gemäß dem Verfahren der Erfindung hergestellten Liposomen, weisen eine Einschlussrate des Proteins/Peptids von mindestens 30 % auf Bei dem erfindungsgemäßen Verfahren erfolgt unter anderem eine Bindung der Proteine oder Peptide an die Membranschicht der sich bildenden Liposomen. Da Innen- und Außenseite der Membranschicht annähernd gleich groß sind, ergibt sich eine statistische Effizienz des Einschlusses von 50%, vor allem unabhängig von der Größe der Liposomen. Bei sehr kleinen Liposomen hat das wegen des hohen Verhältnisses von Lipidfläche zu eingeschlossenem Volumen z.B. die Konsequenz, dass die Konzentration des Stoffes im Innern des Liposoms sehr hoch sein kann, beispielsweise bis zu 100 mg/ml. Die Prozedur des Einschliessens kann so erfolgen, dass die Aktivität der Proteine oder Peptide nicht negativ beeinflusst wird. Beispielsweise konnte für die alkalische Phosphatase ein Erhalt der Funktionalität gezeigt werden.

Mit der Erfindung sind auch Liposomen mit Einschlussraten von mindestens 30% eines Proteins oder Peptides mit einem Durchmesser von 20-500 nm, insbesondere von 30-300 nm, bevorzugt von 50-200 nm und ganz besonders bevorzugt von 70-130 nm herstellbar. Die kleinen Liposomen im Größenbereich von 20-500 nm können insbesondere für Anwendungen im Bereich der Krebstherapie eingesetzt werden, da die kleinen Liposomen selektiv direkt das Tumorgewebe erreichen können, indem sie die Kapillaren im Tumorgewebe durchdringen. Im Tumorgewebe sind die Kapillaren der Blutgefäße permeabler als in gesunden Geweben. Kleine Liposomen können in den Kapillaren die Blutbahn verlassen und sich vorteilhafterweise im Tumorgewebe ansammeln. Die kritische Größe für diesen Mechanismus liegt vorteilhafter Weise im Bereich unter 500 nm, insbesondere unter 300 nm, bevorzugt unter 200 nm und ganz besonders bevorzugt unter 130 nm. Die kleinen Liposomen, beispielsweise im Bereich von unter 130 nm bis 200 nm, können vorteilhafter Weise durch reversible Wechselwirkung von Membran und einzuschließender Substanz effektiv beladen werden, so dass die Substanzen in hoher Konzentration selektiv ins Tumorgewebe gelangen.

Bevorzugt sind die Liposomen unilamellar. Vorteilhafterweise können die durch eine bimolekulare Schicht von Lipiden eingeschlossenen Substanzen schneller freigesetzt werden, als dies beispielsweise bei ogliolamellaren Liposomen möglich ist. Innerhalb des Tumorgewebes können so beispielsweise die Proteine und Peptide in sehr kurzer Zeit freigesetzt werden.

Die Liposomen können zur Herstellung eines Arzneimittels zur Diagnose und/oder Behandlung von Krebserkrankungen verwendet werden. Insbesondere die Liposomen mit Einschlussraten von mindestens 30% eines Proteins Peptides oder einer anderen Substanz mit einem Durchmesser von 20-500 nm, insbesondere von 30-300 nm, bevorzugt von 50-200 nm und ganz besonders bevorzugt von 70-130 nm können mit Vorteil verwendet werden, um Arzneimittel für die Diagnose und/oder Behandlung von Tumoren herzustellen. Die kleinen Liposomen erreichen vorteilhafterweise selektiv direkt das Tumorgewebe, indem sie die Kapillaren im Tumorgewebe durchdringen. Durch die Auswahl geeigneter Größen kann insbesondere das passive Targeting, beispielsweise als Immuntargeting, moduliert werden. Dem Fachmann sind geeignete Cargos bekannt, die zur Diagnose und/oder Behandlung von Krebserkrankungen in die Liposomen eingeschlossen werden können. Selbstverständlich kann auch vorgesehen sein, dass Verbindungen, wie beispielsweise Antikörper, die zur Diagnose oder Behandlung von Krebserkrankungen eingesetzt werden können, in die äußere und/oder innere Membran der Liposomen eingebaut werden.

Die Liposomen können auch als Mikroreaktionsraum in der Enzymkatalyse verwendet werden. Durch die Einkapselung beispielsweise von Enzymen in die Liposomen werden die Enzyme in einen reaktionsraumbegrenzten Zustand versetzt. Vorteilhafterweise können die in die Liposomen eingeschlossenen Enzyme so in lokal höheren Konzentrationen und insbesondere in kontinuierlichen Durchflusssystemen eingesetzt werden.

Durch den Einschluss insbesondere von Enzymen in die Liposomen werden die Enzyme immobilisiert. Unter Immobilisierung werden alle Methoden verstanden, die der Einschränkung der Beweglichkeit und Löslichkeit dienen. Bei den immobilisierten Enzymen kann es sich z.B. um Enzyme handeln, welche an Kompartimente eines Mikroorganismus oder an einen vollständig erhaltenen vitalen oder getrockneten Mikroorganismus gebunden sind, im Sinne der Erfindung kann immobilisiert jedoch auch bedeuten, dass das Enzym alleine oder das Enzym in Verbindung mit Kompartimenten an die Liposomen, die hier als Träger fungieren können, gebunden sind. Von Bedeutung ist, dass die Liposomen mit einem Substrat so in Berührung kommen können, dass die gewünschten enzymatischen Reaktionen ablaufen. Die enzymatische Reaktion kann dabei so ablaufen, dass die umzusetzenden Substrate in die Liposomen durch die Membran diffundieren und innerhalb der Liposomen die entsprechende Umsetzungsreaktion stattfindet. Hierbei ist es z.B möglich, dass die entstehenden Reaktionsprodukte in den Liposomen verbleiben oder aus den Liposomen heraus diffundieren.

Das erfindungsgemäße Verfahren führt zu einer guten Einschlusseffizienz, die überraschend unabhängig von der Teilchengröße und Lamellarität ist. Das Verfahren ist damit in besonderem Maße zu einer Verpackung von biologisch wirksamen Proteinen oder Peptiden in kleine Liposomen geeignet. Das Verfahren arbeitet unter schonenden, physiologischen Bedingungen und trägt damit zum Erhalt der biologischen Aktivität des Cargos bei.

Anschließend wird die Erfindung an Ausführungsbeispielen näher erläutert.

### Beispiele

### Beispiel 1

### Test zur Ermittlung geeigneter Bindungs- und Ablösungsbedingungen

Die Bindungseigenschaften von alkalischer Phosphatase (AP) wurden an einer Reihe von Lipidformulierungen durch das Auftreten von Aggregaten zwischen beiden Komponenten untersucht. Dazu wurde AP mit einer Endkonzentration von 200 µg/ml mit Liposomen aus 70% Phosphatidylcholin und 30% Cholesteryl-N-(Trimethylammonioethyl)-carbamat inkubiert und die entstehenden Aggregate durch Messung des Streulichts charakterisiert. In einem Puffer aus 10 mM Tris(hydroxymethyl)-aminomethan pH 8,0 bindet AP an diese Liposomen und bildet Aggregate. Durch Zusatz von mindestens 100 mM NaCl läßt sich die Aggregatbildung aufheben. Werden Liposomen aus 90% Phosphatidylcholin und 10% Cholesterolhemisuccinat verwendet, so findet keine Aggregation statt.

Aus der Aggregatbildung zwischen Liposomen und Proteinen kann auf geeignete Bedingungen für einen Einschluss geschlossen werden. Für den Test werden schon fertig gebildete Liposomen verwendet, die Reaktion läuft daher nur an der Außenseite ab. Ein solches Vorgehen ermöglicht ein effizientes Screening, da einmal hergestellte Liposomen unter vielen Bedinungen (Salz, pH, Proteinmenge) getestet werden können.

### Beispiel 2

### Einschluss von Alkalischer Phosphatase (AP)

1 mg AP werden in 10 ml Tris(hydroxymethyl)aminomethan (10 mM, pH 8,0) gelöst und 7,1 mg Phosphatidylcholin und 2,2 mg Cholesteryl-N-(Trimethylammonioethyl)-carbamat werden in 100 µl Ethanol suspendiert. Beide Komponenten werden unter Rühren vereinigt und die entstehende Suspension wird dreimal eingefroren und aufgetaut. Die Probe wird für 3 min mit Ultraschall behandelt, anschließend wird die NaCl-Konzentration auf 300 mM eingestellt. Die so hergestellten Liposomen haben eine Größe von 160 nm. Nicht eingeschlossenes Enzym wurde durch Flotation im Sucrosegradienten, wie in Beispiel 3 beschrieben, abgetrennt. 35% des eingesetzten Enzyms werden in den Liposomen gefunden. Mit einem konventionellen Einschluss würde man unter diesen Bedingungen nur 0,5 % des Enzym in den Liposomen finden.

### Beispiel 3

### Erzeugung verschiedener Oberflächenladungen

Liposomen aus Phosphatidylcholin wurden mit Stearinsäure oder Stearylamin dotiert, um eine unterschiedliche Oberflächenladung zu erzeugen. Als einzuschließender Stoff wurde Rinderserumalbumin verwendet.

Drei Ansätze enthielten
1) 400 mg Phosphatidylcholin (neutral)
2) 400 mg Phosphatidylcholin und 7.5mg Stearinsäure (sauer)
3) 400 mg Phosphatidylcholin und 7.5mg Stearylamin (basisch).

Alle drei Gemische wurden in je 1ml Ethanol gelöst.

### a) Lösen des Proteins in einem geeigneten Medium

3*40mg Rinderserumalbumin wurden in jeweils 40ml einer Lösung von 10mM HEPES pH 7.0 gelöst.

### b) Zugabe von membranbildenden Substanzen

Je eine der ethanolischen Lipidlösungen wurde schnell unter Rühren zu einer Lösung des Rinderserumalbumins gegeben:

Dabei bilden sich wenig definierte Liposomen mit einer breiten Größenverteilung. Die erhaltenen Liposomen wurden anschließend mittels Hochdruckhomogenisierung bei 800bar zerkleinert.

Wegen der unterschiedlichen Ladungen von Protein und Liposom kommt es bei der Stearylamin enthaltenden Probe zur Aggregation und schließlich zur Flockung des Kolloids.

### c) Abtrennung von Protein, das sich an der Außenseite der Liposomen befindet

Allen Proben wird jetzt soviel NaCl zugesetzt, dass die Endkonzentration der Lösungen bei 150mM liegt. Die Flocken lösen sich wieder auf und alle Proben bilden stabile Kolloide.

### d) Gewinnung der Liposomen aus der Lösung

Zu analytischen Zwecken wurden die Liposomen aus der Lösung durch Flotation im Schwerefeld einer Ultrazentrifuge gewonnen. Es wurden folgende Einschlusseffizienzen erreicht:

| 1 Liposom | Durchmesser der Liposomen | µg_{Protein} /mg_{Lipid} | % Einschluss |
|---|---|---|---|
| 1)- neutral | 245 nm | 10.0 | 8.5 |
| 2)- sauer | 210 nm | 10.8 | 9.1 |
| 3)- basisch | 140 nm | 65.6 | 55.6 |

## Patentansprüche

1. Verfahren zur Verkapselung von Proteinen oder Peptiden in Liposomen **dadurch gekennzeichnet, dass**
a) das zu verkapselnde Protein oder Peptid in einem wässrigen Puffermedium gelöst wird,
b) Lösungen membranbildender und/oder membranständiger Substanzen zugesetzt werden, die ausgewählt werden aus
- geladenen amphiphilen Verbindungen,
- thiogruppenhaltigen amphiphilen Verbindungen,
- amphiphilen Derivaten der Trinitriloessigsäure und/oder der Iminodiessigsäure
und
der pH-Wert des wässrigen Puffermediums, so eingestellt wird, dass das Protein oder Peptid eine ionische Bindung zu der entstehenden Membran ausbildet,
oder
durch Disulfidbrücken kovalente Bindungen ausgebildet werden,
oder
chelatisierende Interaktionen ausgebildet werden,
c) Protein oder Peptid, das sich an der Aussenseite der gebildeten Liposomenmembran befindet, durch Erhöhung der Ionenstärke des Mediums, Veränderung des pH-Wertes, Zugabe von Reduktionsmitteln und/oder lytischen Enzymen abgetrennt wird und
d) die Liposomen aus der Lösung gewonnen werden.

2. Verfahren nach Anspruch 1,
**dadurch gekennzeichnet, dass**
als membranbildende oder membranständige Substanzen, Substanzen, die mit dem Protein oder Peptid ionische Bindungen, kovalente Bindungen oder Chelatkomplexe ausbilden, in Kombination mit weiteren membranbildenden und/oder membranständigen Substanzen, die keine Bindung mit dem Protein oder Peptid eingehen, eingesetzt werden.

3. Verfahren nach einem der Ansprüche 1 oder 2,
**dadurch gekennzeichnet, dass**
als Substanzen, die ionische Bindungen mit dem Protein oder Peptid eingehen, geladene amphiphile Verbindungen, bevorzugt Alkylcarbonsäuren, Alkylsulfonsäuren, Alkylamine, Alkylammoniumsalze, Phosphorsäureester mit Alkoholen, natürliche sowie synthetische Lipide, wie Phosphatidylglycerol (PG), Phosphatidylserin (PS), Derivate des Phophatidylethanolamins (PE-Derivate) sowie des Cholesterols, Phosphatidsäure, Phosphatidylinositol, Cardiolipin und/oder geladene Sphingolipide eingesetzt werden.

4. Verfahren nach einem der Ansprüche 1 oder 2,
**dadurch gekennzeichnet, dass**
als Substanzen, die kovalente Bindungen mit dem Protein oder Peptid eingehen, solche ausgewählt werden, die mit dem Protein oder Peptid Disulfidbrücken ausbilden, vorzugsweise thiogruppenhaltige Verbindungen, wie z.B. Alkylthiole, Thiocholesterole, Derivate des Phophatidylethanolamins sowie Derivate von Alkylaminen mit freier Thiolfunktion eingesetzt werden.

5. Verfahren nach Anspruch 1 oder 2
**dadurch gekennzeichnet, dass**
als Substanzen, die mit dem zu verkapselnden Protein oder Peptid Chelatkomplexe bilden, amphiphile Derivate der Trinitriloessigsäure und/oder der Iminodiessigsäure eingesetzt werden.

6. Verfahren nach einem der Ansprüche 1 bis 5,
**dadurch gekennzeichnet, dass**
als weitere membranbildende oder membranständige Substanzen Phosphatidylcholin (PC), Phophatidylethanolamin (PE), Cholesterol und/oder neutrale Sphingolipide eingesetzt werden.

7. Verfahren nach einem der Ansprüche 1 bis 6,
**dadurch gekennzeichnet, dass**
das zu verkapselnde Protein oder Peptid vorbehandelt wird, dass es zur Ausbildung von Chelatbindungen oder Disulfidbrücken befähigt ist, wobei diese Vorbehandlung auf chemischem Wege oder durch rekombinante DNA-Techniken erfolgt.

8. Verfahren nach einem der Ansprüche 1 bis 3,
**dadurch gekennzeichnet, dass**
der pH-Wert des wässrigen Mediums, in dem das Protein oder Peptid gelöst wird, so eingestellt wird, dass das Protein oder Peptid eine zu den membranbildenden Substanzen entgegengesetzte Ladung aufweist und die Ionenstärke des Mediums hinreichend gering ist, um die Ausbildung dieser Wechselwirkungen zu erlauben.

9. Verfahren nach Anspruch 4,
**dadurch gekennzeichnet, dass**
der pH-Wert des wässrigen Mediums so eingestellt wird, dass die Ausbildung von Disulfidbrücken zwischen Protein oder Peptid und den membranbildenden oder membranständigen Substanzen erfolgt, und dass ggf. andere Reduktionsmittel entfernt werden.

10. Verfahren nach einem der Ansprüche 2 oder 5;
**dadurch gekennzeichnet, dass**
der pH-Wert des wässrigen Mediums so eingestellt wird, dass die Ausbildung von Chelatbindungen zwischen Protein oder Peptid und den membranbildenden oder membranständigen Substanzen erfolgt, und dass ggf. andere chelatisierende Substanzen entfernt werden.

11. Verfahren nach einem der Ansprüche 1 bis 10,
**dadurch gekennzeichnet, dass**
das an der Außenseite der Liposomen befindliche Protein oder Peptid unter Erhalt seiner Aktivität durch geeignete Waschschritte abgetrennt, aus dem Medium entfernt und ggf. einem erneuten Einsatz zugeführt wird.

12. Verfahren nach Anspruch 11,
**dadurch gekennzeichnet, dass**
das Protein oder Peptid durch lytische Enzyme abgebaut wird.

13. Verfahren nach Anspruch 1 bis 12, **dadurch gekennzeichnet, dass** mindestens 30% eines Proteins oder Peptides in Liposomen mit einem Durchmesser von 20 - 500 nm, insbesondere von 30 - 300 nm, bevorzugt von 50 - 200 nm und ganz besonders bevorzugt von 70 - 130 nm eingeschlossen werden.

14. Verfahren nach Anspruch 13, **dadurch gekennzeichnet, dass** die Liposomen unilamellar sind.

## Claims

1. A method of encapsulating proteins or peptides in liposomes,
**characterized in that**
a) the protein or peptide to be encapsulated is dissolved in an aqueous buffer medium,
b) solutions of membrane-forming and/or membrane-situated substances are added, which are selected from
- charged amphiphilic compounds,
- amphiphilic compounds containing thio groups,
- amphiphilic derivates of trinitriloacetic acid and/or iminodiacetic acid
and
the pH value of the aqueous buffer medium is adjusted in such a way that the protein or peptide undergoes ionic bonds with the membrane being formed,
or
by disulfide bridges covalent bonds are undergone
or
chelating interactions are undergone,
c) the protein or peptide situated on the outside of the liposomal membrane having formed is removed by increasing the ionic strength of the medium, by altering the pH value, by adding reducing agents, and/or by adding lytic enzymes, and
d) the liposomes are recovered from the solution.

2. The method according to claim 1,
**characterized in that**
substances forming ionic bonds, covalent bonds or chelate complexes with said protein or peptide, in combination with other membrane-forming and/or membrane-situated substances not forming bonds with said protein or peptide are used as membrane-forming or membrane-situated substances.

3. The method according to claim 1 or 2,
**characterized in that**
charged amphiphilic compounds, preferably alkylcarboxylic acids, alkylsulfonic acids, alkylamines, alkylammonium salts, phosphoric esters with alcohols, natural and synthetic lipids such as phosphatidyl glycerol (PG), phosphatidyl serine (PS), derivatives of phosphatidyl ethanolamine (PE derivatives) and of cholesterol, phosphatidic acid, phosphatidyl inositol, cardiolipin and/or charged sphingolipids are used as substances forming ionic bonds with said protein or peptide.

4. The method according to claim 1 or 2,
**characterized in that**
substances forming disulfide bridges with said protein or peptide, preferably compounds containing thio groups, such as alkylthiols, thiocholesterols, derivatives of phosphatidyl ethanolamine, as well as derivatives of alkylamines having a free thiol function are used as substances forming covalent bonds with said protein or peptide.

5. The method according to claim 1 or 2,
**characterized in that**
amphiphilic derivatives of trinitriloacetic acid and/or iminodiacetic acid are used as substances forming chelate complexes with the protein or peptide to be encapsulated.

6. The method according to any of claims 1 to 5,
**characterized in that**
phosphatidyl choline (PC), phosphatidyl ethanolamine (PE), cholesterol, and/or neutral sphingolipids are used as additional membrane-forming or membrane-situated substances.

7. The method according to any of claims 1 to 6,
**characterized in that**
the protein or peptide to be encapsulated is subjected to a pretreatment so as to be capable of forming chelate bonds or disulfide bridges, said pre-treatment being performed by chemical means or by using recombinant DNA techniques.

8. The method according to any of claims 1 to 3,
**characterized in that**
the pH value of the aqueous medium wherein said protein or peptide is dissolved is adjusted in such a way that the protein or peptide has a charge opposite to that of the membrane-forming substances and that the ionic strength of the medium is sufficiently low to allow formation of said interactions.

9. The method according to claim 4,
**characterized in that**
the pH value of the aqueous medium is adjusted in such a way that formation of disulfide bridges is effected between said protein or peptide and the membrane-forming or membrane-situated substances, and that other reducing agents are removed, if necessary.

10. The method according to claim 2 or 5,
**characterized in that**
the pH value of the aqueous medium is adjusted in such a way that formation of chelate bonds is effected between said protein or peptide and the membrane-forming or membrane-situated substances, and that other chelating substances are removed, if necessary.

11. The method according to any of claims 1 to 10,
**characterized in that**
the protein or peptide situated on the outside of the liposomes is separated by means of suitable washing steps, while retaining the activity thereof, removed from the medium, and optionally put to use again.

12. The method according to claim 11,
**characterized in that**
the protein or peptide is degraded by lytic enzymes.

13. The method according to claims 1 to 12,
**characterized in that**
at least 30% of a protein or peptide are encapsulated in liposomes with a diameter of 20-500 nm, particularly 30-300 nm, preferably 50-200 nm, and more preferably 70-130 nm.

14. The method according to claim 13,
**characterized in that**
the liposomes are unilamellar.

## Revendications

1. Procédé d'encapsulage de protéines ou de peptides dans des liposomes,
**caractérisé en ce que**
a) on dissous la protéine ou le peptide à encapsuler dans un milieu tampon aqueux,
b) on ajoute des solutions des substances générant une membrane et/ou étant liés à une membrane, qui sont sélectionnées parmi
- des composés amphiphiles chargés,
- des composés amphiphiles contenant des groupements thio,
- des dérivés amphiphiles de l'acide trinitriloacétique et/ou de l'acide iminodiacétique,
et
on ajuste le pH du milieu tampon aqueux de telle sorte que la protéine ou le peptide forme une liaison ionique avec la membrane formée,
ou
que des liaisons covalentes soient formées par des ponts disulfure,
ou
que des interactions chélatantes se forment,
c) on sépare la protéine ou le peptide qui se trouve sur le côté extérieur de la membrane de liposomes formée en augmentant la force ionique du milieu, en modifiant le pH, en ajoutant des agents de réduction et/ou des enzymes lytiques et
d) on extrait les liposomes de la solution.

2. Procédé selon la revendication 1,
**caractérisé en ce**
**qu'**on utilise, en tant que substances générant une membrane ou étant liés à une membrane, des substances qui forment avec la protéine ou le peptide des liaisons ioniques, des liaisons covalentes ou des complexes chélatés, en association avec d'autres substances générant une membrane et/ou étant liés à une membrane qui ne contractent aucune liaison avec la protéine ou le peptide.

3. Procédé selon une des revendications 1 ou 2,
**caractérisé en ce**
**qu'**on utilise, en tant que substances contractant des liaisons ioniques avec la protéine ou le peptide, des composés amphiphiles chargés, de préférence des acides alkylcarboxyliques, des acides alkylsulfoniques, des alkylamines, des sels d'alkylammonium, des esters d'acide phosphorique avec des alcools, des lipides naturels ou synthétiques tels que du phosphatidylglycérol (PG), de la phosphatidylsérine (PS), des dérivés du phosphatidyléthanolamine (dérivés de PE) ainsi que du cholestérol, de l'acide phosphatidique, du phosphatidylinositol, du cardiolipide et/ou des sphingolipides chargés.

4. Procédé selon une des revendications 1 ou 2,
**caractérisé en ce**
**qu'**on choisi, en tant que substances contractant des liaisons ioniques avec la protéine ou le peptide, des substances qui forment avec la protéine ou le peptide des ponts disulfure, de préférence des composés contenant des groupements thio, comme par ex. des alkylthiols, des thiocholestérols, des dérivés du phosphatidyléthanolamine ainsi que des dérivés d'alkylamines comportant une fonction thiol libre.

5. Procédé selon la revendication 1 ou 2,
**caractérisé en ce**
**qu'**on utilise, en tant que substances formant un complexe chélaté avec la protéine ou le peptide à encapsuler, des dérivés amphiphiles de l'acide trinitriloacétique et/ou de l'acide iminodiacétique.

6. Procédé selon une des revendications 1 à 5,
**caractérisé en ce**
**qu'**en tant qu'autres substances générant une membrane et/ou étant liés à une membrane, on utilise de la phosphatidylcholine (PC), du phosphatidyléthanolamine (PE), du cholestérol et/ou des sphingolipides neutres.

7. Procédé selon une des revendications 1 à 6,
**caractérisé en ce**
**qu'**on effectue un traitement préliminaire de la protéine ou du peptide à encapsuler, de telle sorte qu'il soit capable de former des liaisons chélate ou des ponts disulfure, ce traitement préalable étant effectué par voie chimique ou par des techniques d'ADN recombinant.

8. Procédé selon une des revendications 1 à 3,
**caractérisé en ce**
**qu'**on ajuste le pH du milieu aqueux dans lequel la protéine ou le peptide est dissous de telle sorte que la protéine ou le peptide présente une charge opposée vis à vis des substances générant une membrane et que la force ionique soit suffisamment faible pour permettre la formation de ces interactions.

9. Procédé selon la revendication 4,
**caractérisé en ce**
**qu'**on ajuste le pH du milieu aqueux de telle sorte que la formation de ponts disulfure s'effectue entre la protéine ou le peptide et les substances générant une membrane et/ou étant liés à une membrane et qu'éventuellement d'autres agents réducteurs soient éliminés.

10. Procédé selon une des revendications 2 ou 5,
**caractérisé en ce**
**qu'**on ajuste le pH du milieu aqueux de telle sorte que la formation de liaisons chélate s'effectue entre la protéine ou le peptide et les substances générant une membrane et/ou étant liés à une membrane et qu'éventuellement d'autres substances chélatantes soient éliminées.

11. Procédé selon une des revendications 1 à 10,
**caractérisé en ce**
**qu'**on sépare la protéine ou le peptide se trouvant à l'extérieur des liposomes en maintenant son activité par des étapes de lavage appropriées, on l'enlève du milieu et on l'amène éventuellement vers une nouvelle utilisation.

12. Procédé selon la revendication 11,
**caractérisé en ce**
**qu'**on décompose la protéine ou le peptide par des enzymes lytiques.

13. Procédé selon une des revendications 1 à 12,
**caractérisé en ce**
**qu'**on insère au moins 30 % d'une protéine ou d'un peptide dans des liposomes ayant un diamètre de 20 à 500 nm, plus particulièrement de 30 à 300 nm, de préférence de 50 à 200 nm et de manière encore plus préférée de 70 à 130 nm.

14. Procédé selon la revendication 13,
**caractérisé en ce que**
les liposomes sont unilamellaires.
